# EUROPÄISCHE PATENTSCHRIFT

(11) **EP 1 606 606 B1**
(45) Veröffentlichungstag und Bekanntmachung des Hinweises auf die Patenterteilung: **13.07.2011**
(21) Anmeldenummer: 04718922.0
(22) Anmeldetag: 10.03.2004
(51) Int. Cl.: G01N 1/08, A01K 11/00

(54) **GEWEBEBESTANZVORRICHTUNG**
TISSUE PUNCHING DEVICE
Dispositif pour poinçonner un tissu

(30) Priorität: 25.03.2003 DE 10313340
(43) Veröffentlichungstag der Anmeldung: 21.12.2005
(73) Patentinhaber: Helmholtz Zentrum München Deutsches Forschungszentrum für Gesundheit und Umwelt (GmbH), 85764 Neuherberg (DE)
(72) Erfinder: BRIELMEIER, Markus, 85748 Garching (DE); SCHMIDT, Joerg, 80538 München (DE)
(74) Vertreter: Charles, Glyndwr
(86) Internationale Anmeldenummer: PCT/EP2004/002457
(87) Internationale Veröffentlichungsnummer: WO 2004/085999

(56) Entgegenhaltungen:
- EP-A- 1 088 212
- EP-A- 1 219 169
- DE-A- 19 740 429
- FR-A- 2 831 388

## Beschreibung

Die Erfindung betrifft eine Vorrichtung zum Herausstanzen einer Gewebeprobe, insbesondere von Tieren zur Kennzeichnung bzw. Markierung und zur Bestimmung von deren Genotyp.

In der medizinischen Forschung werden zunehmend genetisch veränderte Tiere eingesetzt, die auch als transgene Tiere bezeichnet werden. Bei der Aufzucht dieser Tiere zu Forschungszwecken wird von jedem geborenen Tier der Genotyp ermittelt, damit die gewünschten Merkmalsträger erkannt und für die weitere Zucht verwendet werden. Dies geschieht herkömmlicher Weise durch Analyse von Gewebeproben mit molekularbiologischen Methoden, wie beispielsweise der Polymerase-Kettenreaktion (PCR). Zur Gewinnung der Gewebeprobe wird dabei üblicherweise von dem zu genotypisierenden Tier, bei dem es sich beispielsweise um eine Maus handelt, in der Regel ohne Anästhesie ein Stück des Schwanzes amputiert. Nach Inkubation der Gewebeprobe in einer Enzymlösung und anschließender Isolation der DNA wird mittels molekularbiologischer Verfahren, vornehmlich der Polymerase-Kettenreaktion (PCR), ein charakteristisches Gensegment dieser Probe vervielfältigt und analysiert.

Die molekularbiologischen Methoden zur Gewinnung eines charakteristischen Gensegments der Gewebeprobe sind einerseits sehr empfindlich und für das zu untersuchende- Transgen spezifisch, sind jedoch andererseits aufgrund ihrer Sensibilität äußerst anfällig gegenüber geringsten Verunreinigungen von Restgeweben anderer Tiere. Wird zunächst eine Gewebeprobe von einem transgenen positiven Tier entnommen und anschließend eine Gewebeprobe von einem Tier, das nicht transgen ist, besteht die Gefahr, dass durch die Verwendung der gleichen Vorrichtung zur Probeentnahme transgene positive Gewebeteile verschleppt werden und das nachfolgende nicht transgene Tier irrtümlicher Weise als transgen erkannt wird.

Die DE 197 40 429 A1 beschreibt ein Verfahren und eine Vorrichtung zur Entnahme von biologischen Proben. Mittels einer Zange wird ein Probenkapselverschluss mit einer scharfen Außenkante als Stempel durch das Gewebe gegen den als Matrize wirkenden Probenkapselbehälter gedrückt, so dass dadurch eine Gewebeprobe abgeschert wird. Durch eine runde Nut im Probenkapselbehälter und das entsprechende Gegenstück in dem Probenkapselverschluss werden dabei beide Teile miteinander zu einer Probenkapsel verschlossen, so dass ein unerwünschtes Öffnen verhindert wird. Die DE 197 40 429 A1 beschreibt ferner eine Probenkapsel bei der der Probenbehälter und der dazugehörige Deckel aus einem Teil bestehen und über ein Gelenk miteinander verbunden sind.

Die EP 1 088 212 B1 beschreibt eine Vorrichtung und ein Verfahren zur Gewinnung und Erstaufbereitung von Gewebeproben für die molekulargenetische Diagnostik. Mit Hilfe einer Ohrzange wird ein Probengewinnungsmittel mit einem scharfkantigen vorderen Ende durch das Ohr gestochen und in dem gleichen Arbeitsgang in den Probeaufnahmebehälter gedrückt. Durch ein Befestigungsmittel wird das Probengewinnungsmittel in dem Probeaufnahmebehälter fixiert.

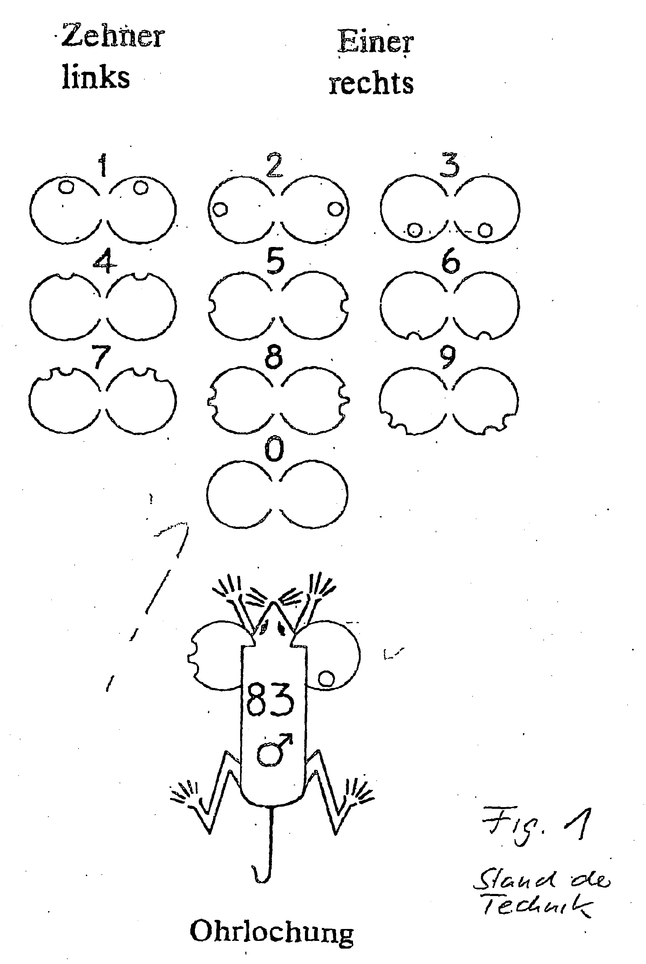

Damit die Gewebeproben den richtigen Tieren zugeordnet werden können, müssen die zu untersuchenden Tiere gekennzeichnet werden. Dies geschieht üblicherweise dadurch, dass man eine Ohrlochung vornimmt, d.h. die Ohren des zu untersuchenden Tieres werden durch entsprechende Stanzung gekennzeichnet. Figur 1 zeigt ein Beispiel für die dabei verwendete Kodierung zur Kennzeichnung des zu untersuchenden Tieres. Jeder Zahl ist dabei ein entsprechendes Stanzmuster zugeordnet, wie in Figur 1 dargestellt. Beispielsweise wird die Zahl 1 durch eine runde Stanzung an der Vorderseite des Ohres dargestellt, die Zahl 2 durch eine Stanzung an der Seite des Ohres und die Zahl 3 durch eine Stanzung an der Hinterseite des Ohres. Die Zahlen 4 bis 6 werden in entsprechender Weise durch Einkerbungen bzw. halbkreisförmige Einstanzungen dargestellt. Die Zahlen 7 bis9 werden durch Doppelstanzung bzw. Doppelkerben kodiert. Weist das Ohr keine Stanzung auf, steht dies für die Zahl 0. Per Konvention dient das linke Ohr zur Kennzeichnung von Vielfachen der Zahl 10 und das rechte Ohr zur Kennzeichnung gewöhnlicher Zahlwerte. Bei dem in Figur 1 dargestellten Beispiel handelt es sich somit um das 83. Tier der zu untersuchenden Gruppe.

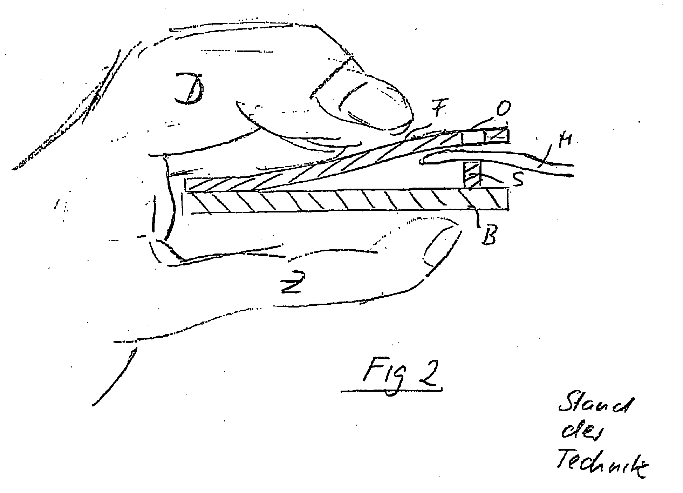

Figur 2 zeigt eine herkömmliche Vorrichtung zur Markierung von Mäusen entsprechend dem in Figur 1 angegebenen Kodierschema. Die Stanzvorrichtung besteht aus einer Bodenplatte B auf der eine zylinderförmige Stanzeinrichtung S angebracht ist. Die Bodenplatte trägt ferner eine Metall-Blattfeder F in der eine Stanzöffnung O vorgesehen ist. Die Stanzfeder F kann manuell durch Handdruck abgesenkt werden, wobei die Stanze S ganz genau in die Stanzöffnung O eingeführt wird. Bei Markierung der Maus wird das linke oder rechte Mauseohr M zwischen die Stanzfeder F und den Stanzzylinder S geschoben und anschließend die Stanzfeder F manuell nach unten gedrückt, so dass ein Gewebestück des Mauseohrs M durch die Öffnung O herausgestanzt wird. Die in Figur 2 dargestellte Stanzvorrichtung zur Markierung von Mäusen nach dem Stand der Technik besteht aus einem Metall, das in einfacher Weise hitzesterilisierbar ist. Durch die Verwendung von Metall als Werkstoff ist es möglich die in Figur 2 dargestellte Stanzvorrichtung zur Vornahme einer Vielzahl von Markierungen einzusetzen. Alternativ kann die Stanzeinrichtung S auch an der Blattfeder, und die Stanzöffnung O in der Bodenplatte angebracht sein.

Die in Figur 2 dargestellte Stanzvorrichtung zur Markierung von Ohren eignet sich nicht zum Herausstanzen von Gewebeproben. Da die Ohren der zu untersuchenden Tiere, eine vorgegebene Größe aufweisen, können die daraus gestanzten Markierungen ebenfalls eine gewisse Größe nicht überschreiten. Die Öffnung O der in Figur 2 dargestellten herkömmlichen Markiervorrichtung weist einen Durchmesser von etwa zwei Millimetern auf. Dementsprechend ist das aus dem Ohr M herausgestanzte Gewebestück ebenfalls relativ klein und weist maximal einen Durchmesser von zwei Millimetern auf. Zur Betätigung der Markiervorrichtung gemäß Figur 2 wird diese üblicherweise zwischen einem Daumen D und einem Zeigefinger Z der Bedienperson geklemmt. Bei entsprechender Handhabung der Markiervorrichtung fällt das herausgestanzte Gewebestück durch die Öffnung O nach oben oder alternativ nach unten und kann durch einen Aufnahmebehälter zur weiteren Analyse aufgefangen werden. Allerdings ist die Betätigung der Markiervorrichtung zu diesem Zweck sehr umständlich, da mit einer Hand die Markiervorrichtung gemäß Figur 2 bedient wird und mit der anderen Hand die zu untersuchende Maus zur Markierung von deren Ohr festgehalten werden muss. Es ist daher für die Bedienperson in der Regel nicht möglich die herausgestanzte Markierung als Gewebeprobe in einem darunter befindliches Aufnahmegerät zur weiteren Analyse aufzufangen.

Ein weiterer gravierender Nachteil herkömmlicher Stanzgeräte besteht darin, dass es sich bei der in Figur 2 dargestellten Markiervorrichtung zur Markierung von Mäusen um ein Mehrfachwerkzeug handelt, d.h. die Stanzvorrichtung wird zur Markierung einer Vielzahl von unterschiedlichen zu untersuchenden Tieren eingesetzt. Es kann daher vorkommen, dass Gewebereste oder Blutzellen an der Stanze S bzw. der Öffnung O der Markiervorrichtung hängen bleiben und somit das Untersuchungsergebnis der nachfolgend markierten Tiere verfälschen.

Es ist daher die Aufgabe der vorliegenden Erfindung eine Vorrichtung zum Herausstanzen einer Gewebeproben zu schaffen, bei der die herausgestanzte Gewebeprobe sicher in einem Untersuchungsgefäß aufgefangen wird und keine Verfälschung von Untersuchungsergebnissen aufgrund verschleppter Gewebeproben auftreten.

Diese Aufgabe wird erfindungsgemäß durch eine Vorrichtung mit den in Patentanspruch 1 angegebenen Merkmalen gelöst.

Die Erfindung schafft eine Vorrichtung zum Herausstanzen einer Gewebeprobe mit einem Aufnahmebehälter zur Aufnahme der Gewebeprobe, einem in den Aufnahmebehälter einsetzbaren Behälterverschluss zum Verschließen des Aufnahmebehälters, wobei der Behälterverschluss eine Stanzöffnung aufweist und mit einem mit dem Behälterverschluss verbundenen Bügel, der eine in die Stanzöffnung passgenau absenkbare Stanze trägt.

Die Grundidee der Erfindung besteht darin, eine Vorrichtung zu schaffen, bei der in einem Arbeitsschritt sowohl eine Markierung des zu untersuchenden Tieres erfolgt und gleichzeitig dem Tier eine zugehörige Gewebeprobe entnommen wird.

Dies hat den besonderen Vorteil, dass einerseits ein Arbeitsschritt eingespart werden kann und zusätzlich eine Fehlzuordnung zwischen dem markierten Tier und dessen entnommener Gewebeprobe weitestgehend ausgeschlossen ist.

Ein weiterer Vorteil der erfindungsgemäßen Vorrichtung besteht darin, dass dem Tier neben der Markierung im Ohr nicht noch zusätzlich eine Gewebeprobe durch Amputieren der Schwanzspitze entnommen werden muss. Dies stellt einen Beitrag zum Tierschutz dar.

Die erfindungsgemäße Vorrichtung zum Herausstanzen einer Gewebeprobe ist ein Einmalwerkzeug, d.h. die erfindungsgemäße Stanzvorrichtung wird bei einem einzigen zu untersuchenden Tier eingesetzt.

Bei einer bevorzugten Ausführungsform der erfindungsgemäßen Vorrichtung bestehen der Behälterverschluss und der Bügels aus Kunststoff.

Die Stanze wird vorzugsweise an einem distalen Ende des Bügels angebracht und ist gegen eine Federkraft des Bügels durch einen manuell aufgebrachten mechanischen Druck in die Stanzöffnung des Behälterverschlusses absenkbar.

Bei einer besonders bevorzugen Ausführungsform ist zusätzlich eine Halteeinrichtung vorgesehen, in die der durch den Behälterverschluss verschlossene Aufnahmebehälter eingehängt wird.

Diese Halteeinrichtung weist vorzugsweise eine manuell betätigbare Druckfeder auf, durch die der mechanische Druck auf den Bügel aufgebracht werden kann.

Ein Vorteil der Halteeinrichtung besteht darin, dass hierdurch die manuelle Betätigung der erfindungsgemäßen Stanzvorrichtung erheblich erleichert wird.

Bei einer bevorzugten Ausführungsform der erfindungsgemäßen Vorrichtung federt die durch den Bügel getragene und in die Stanzöffnung passgenau abgesenkte Stanze durch die Federkraft des Bügels zur erneuten Betätigung der erfindungsgemäßen Stanzvorrichtung von dem Behälterverschluss zurück, wenn der durch die Druckfeder manuell aufgebrachte mechanische Druck gering dosiert ist.

Bei einer bevorzugten Ausführungsform der erfindungsgemäßen Stanzvorrichtung bleibt die durch den Bügel getragene und in die Stanzöffnung passgenau abgesenkte Stanze in dem Behälterverschluss zu dessen Versiegelung stecken, wenn der durch die Druckfeder manuell aufgebrachte mechanische Druck erst nach einer längeren Zeit beendet oder bei der letzten Stanzprobe eines Tieres ein stärkerer Druck ausgeübt wird.

Dabei ist die durch den Bügel getragene Stanze vorzugsweise über eine Sollbruchstelle mit dem Bügel verbunden.

Bei einer bevorzugten Ausführungsform besteht die Feder der Haltevorrichtung aus Metall.

Bei einer alternativen Ausführungsform besteht die Feder der Haltevorrichtung aus Kunststoff.

Der Aufnahmebehälter der erfindungsgemäßen Stanzvorrichtung ist vorzugsweise zylinderförmig, wobei die Stanzöffnung des Behälterverschlusses exzentrisch zu der Symmetrieachse des Aufnahmebehälters angeordnet ist.

Der Aufnahmebehälter ist vorzugsweise transparent.

Bei einer besonders bevorzugten Ausführungsform der erfindungsgemäßen Stanzvorrichtung ist in dem Aufnahmebehälter bereits eine Reaktionsflüssigkeit für die herausgestanzte Gewebeprobe abgefüllt.

Dies bietet den besonderen Vorteil, dass der Arbeitsschritt zum Einfüllen der Reaktionsflüssigkeit nach der Stanzung nicht mehr notwendig ist.

Bei einer besonders bevorzugten Ausführungsform der erfindungsgemäßen Stanzvorrichtung handelt es sich bei dem Aufnahmebehälter um ein Eppendorf-Gefäß oder um ein diesem Gefäßtyp ähnliches Gefäß.

Im weiteren werden bevorzugte Ausführungsformen der erfindungsgemäßen Vorrichtung zum Herausstanzen einer Gewebeprobe unter Bezugnahme auf die beigefügten Figuren zur Erläuterung erfindungswesentlicher Merkmale beschrieben.

Es zeigen:
- Figur 1: ein Kodierschema zur Markierung von Versuchstieren nach dem Stand der Technik;
- Figur 2: eine Markiervorrichtung zur Markierung von Ver- suchstieren nach dem Stand der Technik;
- Figur 3: eine erste Ausführungsform der erfindungsgemäßen Vor- richtung zum Herausstanzen einer Gewebeprobe;

- Figur 4: eine bevorzugte Ausführungsform der erfindungsgemäßen Vorrichtung zum Herausstanzen einer Gewebeprobe;
- Figur 5: eine Ansicht der in Figur 4 dargestellten Stanzvor- richtung von oben;
- Figur 6a, Figur 6b: eine Aufnahmevorrichtung zur Aufnahme mehrerer erfin- dungsgemäßer Stanzvorrichtungen.

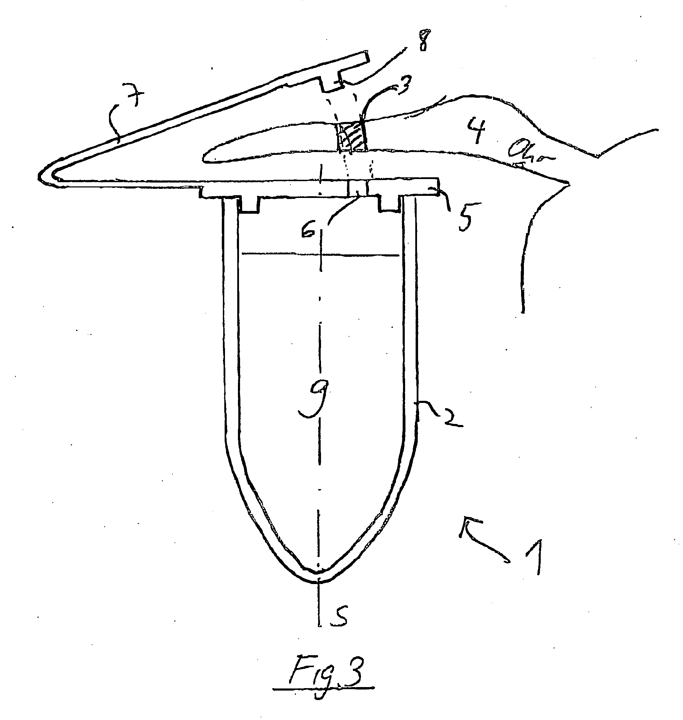

Figur 3 zeigt eine bevorzugte Ausführungsform einer erfindungsgemäßen Stanzvorrichtung 1 zum Herausstanzen einer Gewebeprobe. Die Stanzvorrichtung 1 gemäß der Erfindung weist einen Aufnahmebehälter 2 auf. Der Aufnahmebehälter 2 dient zur Aufnahme einer Gewebeprobe 3, die aus einem Ohr 4 eines Versüchtiers herausgestanzt wird. Der Aufnahmebehälter 2 ist nach oben offen, wobei in die Öffnung ein Behälterverschluss 5 zum Verschließen des Aufnahmebehälters 2 einsetzbar ist. Der Behälterverschluss 5 ist mit dem Aufnahmebehälter 2 lösbar verbunden. Beispielsweise wird der Behälterverschluss 5 in die Öffnung des Aufnahmebehälters 2 hineingedrückt. Bei einer besonderen Ausführungsform schnappt der Behälterverschluss 5 in den Aufnahmebehälter 2 ein. Bei dem Aufnahmebehälter 2 handelt es sich vorzugsweise um ein Eppendorf-Gefäß.

In den Aufnahmebehälter 2 ist bei einer besonders bevorzugten Ausführungsform bereits eine Reaktionsflüssigkeit für die herausgestanzte Gewebeprobe abgefüllt. Bei dieser Reaktionsflüssigkeit handelt es sich vorzugsweise um eine Enzymlösung zur DNA-Isolierung.

Der Behälterverschluss 5 weist eine Stanzöffnung 6 auf, die vorzugsweise rund ist. Der in Figur 3 dargestellte Aufnahmebehälter 2 ist vorzugsweise zylinderförmig und symmetrisch zu einer Symmetrielinie S. Die Stanzöffnung 6 liegt vorzugsweise nicht auf der Symmetrielinie S, sondern exzentrisch dazu, wie in Figur 3 dargestellt. Hierdurch ist es möglich auch Stanzlöcher am hinteren Ende des Ohres 4 anzubringen.

An dem Behälterverschluss 5 befindet sich ein Bügel 7, der eine in die Stanzöffnung 6 passgenau absenkbare Stanze 8 trägt. Der Behälterverschluss 5 und der damit integral verbundene Bügel 7 besteht vorzugsweise aus Kunststoff, insbesondere aus Polystyrol. Der Durchmesser der Stanzöffnung 6 und der zylinderförmigen Stanze 8 beträgt vorzugsweise 2 mm ± 0,5 mm. Hierdurch ist die Stanzvorrichtung 1 zur Kennzeichnung von Mause- oder Rattenohren entsprechend Figur 1 geeignet. Aufgrund des relativ geringen Durchmessers der Stanzöffnung 6 fließt die in dem Aufnahmebehälter 2 enthaltene Reaktionsflüssigkeit 9 aufgrund von deren Oberflächenspannung aus dem Aufnahmebehälter 2 nicht heraus, selbst wenn die Stanzvorrichtung 1 beim Herausstanzen der Gewebeproben derart gehalten wird, dass die Stanzöffnung 6 nach unten zeigt. Bei einer ersten Ausführungsform der erfindungsgemäßen Stanzvorrichtung 1 bilden der Behälterverschluss 5 einschließlich Bügel 7 ein erstes Bauteil, das in den Aufnahmebehälter 2 als separates Bauteil einsetzbar ist. Dies ermöglicht der Bedienperson eine zu seinen speziellen Forschungszwecken passende Reaktionsflüssigkeit 9 in den Aufnahmebehälter 2 einzufüllen und anschließend die Gewebeprobe herauszustanzen.

Bei einer alternativen Ausführungsform wird der Behälterverschluss 5 und dessen Bügel 7 mit dem Aufnahmebehälter 2 fest verbunden, nachdem der Aufnahmebehälter 2 mit einer vorgegebenen Reaktionsflüssigkeit gefüllt ist. Bei dieser Ausführungsform wird die Reaktionsflüssigkeit bereits von dem Hersteller in den Aufnahmebehälter 2 abgefüllt und an den Kunden ausgeliefert. Dies hat für die Bedienperson den Vorteil, dass die Reaktionsflüssigkeit nicht noch manuell in den Aufnahmebehälter 2 abgefüllt werden muss. Dabei wird vorzugsweise der Behälterverschluss durch Herunterdrücken des Bügel 7 geschlossen ausgeliefert, d.h. die Stanze 8 beschließt die Stanzöffnung 6. Diese Ausführung bietet den besonderen Vorteil, dass die in dem Aufnahmebehälter 2 abgefüllte Reaktionsflüssigkeit 9 vor Verunreinigungen geschützt ist.

Zur Gewinnung einer Gewebeprobe nimmt die Bedienperson die Stanzvorrichtung 1 in die Hand und klemmt den V-förmigen Bügel 7 zwischen zwei Finger. Anschließend wird das Ohr 4 des Versuchtiers zwischen die beiden Schenkel des Bügels 7 geführt und die Stanze 8 manuell in die Stanzöffnung 6 gedrückt. Die zylinderförmige Stanzeinrichtung 8 weist vorzugsweise scharfe Kanten auf, welche in das Gewebe 4 einschneiden, so dass eine zylinderförmige Gewebeprobe 3 in den Aufnahmebehälter 2 fällt. Nach Inkubation des Gewebestücks 3 in der Enzymlösung 9 und anschließender Isolation der DNA wird mittels molekularbiologischer Methoden, beispielsweise mittels PCR ein charakteristisches Gensegment aus der Gewebeprobe 3 vervielfältigt und anschließend analysiert. Hierzu wird vorzugsweise eine Flüssigkeitsprobe aus dem Aufnahmebehälter 2 herauspipetiert.

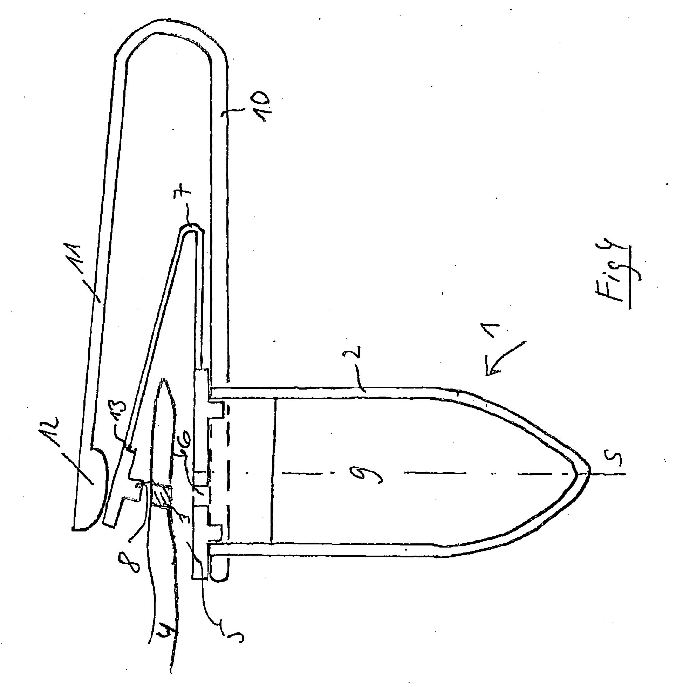

Figur 4 zeigt eine besonders bevorzugte Ausführungsform zur besseren, sichereren und gefühlvolleren Handhabung der erfindungsgemäßen Stanzvorrichtung 1. Bei der in Figur 4 dargestellten Ausführungsform weist die Stanzvorrichtung 1 zusätzlich eine Halteeinrichtung 10 auf, in die der durch den-Behälterverschluss 5 verschlossene Aufnahmebehälter 2 eingehängt wird. Diese Halteeinrichtung 10 ist bevorzugt aus Metall oder aus Kunststoff. Die Halteeinrichtung 10 weist eine manuell betätigbare Druckfeder 11 auf, deren Endstück 12 gegen das Ende des Bügels 7 manuell gedrückt werden kann. Bei dem Aufnahmebehälter 2 handelt es sich vorzugsweise um ein sogenanntes Eppendorf-Gefäß. Diese Eppendorf-Gefäße sind herkömmlicherweise in ihrer geometrischen Abmessung relativ klein. Die Haltereinrichtung 10 ist derart ausgebildet, dass sie eine Größe aufweist, die eine einfache Handhabe durch eine Bedienperson erlaubt. Die Bedienperson übt eine Druckkraft auf die Feder 11 aus. Diese Kraft wird durch die Auswölbung 12 auf die Stanze 8 übertragen, die das Gewebestück 3 aus dem Ohr herausstanzt. Das herausgestanzte Gewebestück 3 fällt durch die Öffnung 6 in die Reaktionsflüssigkeit 9 des Aufnahmebehälters 2. Die Feder 11 besteht vorzugsweise aus Metall. Bei einer alternativen Ausführungsform besteht die Feder 11 aus Kunststoff.

Ein besonderer Vorteil der in Figur 4 dargestellten Ausführungsform besteht darin, dass kein Kontakt zwischen der Hand der Bedienperson und dem Gewebestück 3 besteht. Hierdurch wird sichergestellt, dass Stoffreste, die sich auf der Hand der Bedienperson befinden, das Gewebestück 3 nicht verunreinigen.

Bei einer bevorzugten Ausführungsform der erfindungsgemäßen Stanzvorrichtung 1 wird die durch den Bügel 7 getragene und in die Stanzöffnung 6 passgenau abgesenkte Stanze 8 durch die Federkraft des Bügels 7 zur erneuten Betätigung nach dem Stanzvorgang zurückgefedert. Dies geschieht insbesondere dann, wenn der durch die Druckfeder 11 manuell aufgebrachte mechanische Druck gering ist. Drückt die Bedienperson die Druckfeder 11 für eine längere Zeit oder mit höherem Druck gegen den abgesenkten Bügel 7 bleibt bei einer bevorzugten Ausführungsform die abgesenkte Stanze 8 in dem Behälterverschluss 6, zu dessen Versiegelung stecken. Dabei weist der Bügel 7 bei einer besonders bevorzugten Ausführungsform eine Sollbruchstelle 13 auf. Wird beispielsweise mit der erfindungsgemäßen Stanzvorrichtung 1 der in Figur 1 dargestellten Maus 83 eine Gewebeprobe entnommen, ist diese Maus durch drei Stanzvorgänge zu markieren. Bei diesem Stanzvorgang ist an dem linken Ohr eine acht zu markieren und an dem rechten Ohr eine drei. Hierzu wird die erfindungsgemäße Stanzvorrichtung 1 zunächst zweimal an den Rand des linken Ohrs angelegt und zwei halbkreisförmige Ausstanzungen bzw. Einkerbungen vorgenommen. Anschließend wird mittels der erfindungsgemäßen Stanzvorrichtung 1 das rechte Ohr als drei markiert. Die drei Stanzvorgänge können in einer beliebigen Reihenfolge durchgeführt werden. Bei dem dritten und letzten Stanzvorgang drückt die Bedienperson die Feder 11 solange oder mit derart hohem Druck gegen die Stanze 8 bis diese in der Stanzöffnung 6 stecken bleibt und den Aufnahmebehälter 2 vollständig verschließt. Bei dem ersten und zweiten Stanzvorgang übt die Bedienperson einen Druck auf die Feder 11 aus, so dass Bügel 7 für eine weitere Betätigung zurückfedern kann. Erst beim dritten Stanzvorgang bricht die Sollbruchstelle 13 und die Stanze 8 bleibt in dem Behälterverschluss stecken. Anschließend wird ein neuer Aufnahmebehälter 2 zum Markieren des nächsten Tieres eingehängt.

Aus dem in Figur 1 dargestellten Versuchtier 83 werden drei Gewebestücke herausgestanzt, die in die Reaktionsflüssigkeit 9 hineinfallen. Die Markierung und die Gewinnung einer Gewebeprobe aus einem Untersuchungstier erfolgt erfindungsgemäß in einem Arbeitsschritt. Auch das Einfüllen der Reaktionsflüssigkeit 9 in den Aufnahmebehälter 2 kann bei der erfindungsgemäßen Vorrichtung entweder vom Hersteller durchgeführt und so dem Experiment angeboten werden. Die Reaktionsflüssigkeit wird von der Bedienperson entsprechend ihren besonderen Erfordernissen im Labor eingefüllt, bevor sie in den Raum mit den Versuchstieren gelangt.

Der Aufnahmebehälter 2 ist vorzugsweise transparent, so dass die Bedienperson kontrollieren kann, ob er die gewünschte Enzymlösung enthält und ob die entnommene Gewebeprobe 3 in die Reaktionsflüssigkeit 9 hineingefallen ist. Nachdem die Gewebeprobe 3 in den Aufnahmebehälter 2 gefallen ist, wird mittels eines Stiftes die zugehörige Nummer des Versuchtiers auf den Aufnahmebehälter 2 geschrieben. Bei dem in Figur 1 dargestellten Beispiel schreibt die Bedienperson zum Beispiel die Nummer 83 mit Hilfe eines Stiftes auf den Aufnahmebehälter 2.

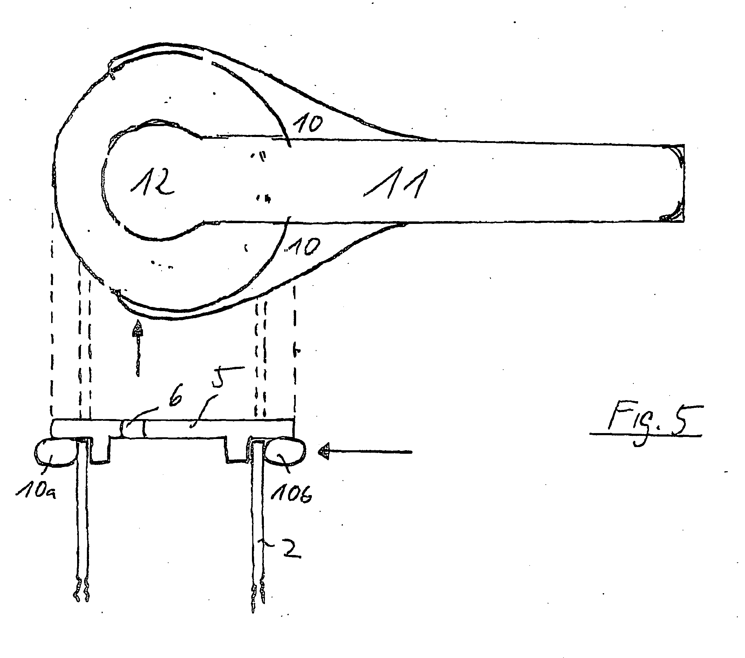

Figur 5 zeigt in Figur 4 dargestellte Stanzvorrichtung von oben und in Schnittansicht von vorne.

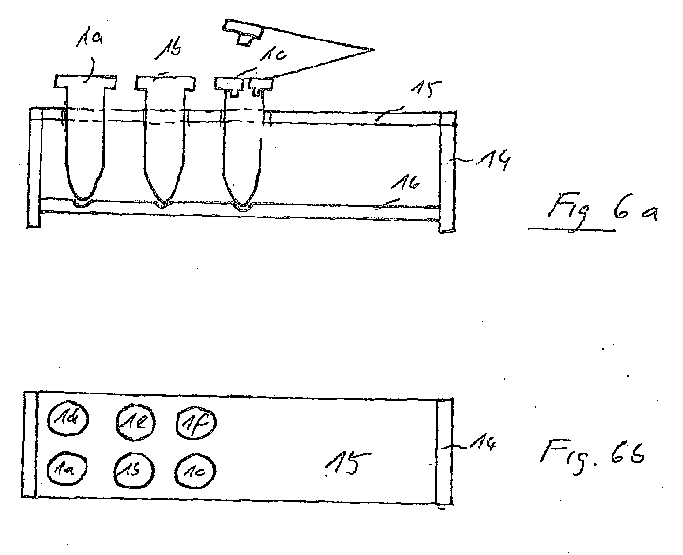

Figuren 6a, 6b zeigen einen Ständer 14 zur Aufnahme mehrerer Stanzvorrichtungen 1 gemäß der Erfindung. Figur 6a zeigt eine Schnittansicht und Figur 6b eine Ansicht von oben. Bei dem in Figur 6 dargestellten Beispiel werden sechs erfindungsgemäße Stanzvorrichtungen 1 gemäß Figur 3 in dem Ständer 14 gehalten. Der Ständer 14 weist eine erste Platte 15 auf, in der Öffnungen angebracht sind, um die Aufnahmebehälter 2 aufzunehmen. Eine zweite Platte 16 hält die Spitzen der Aufnahmebehälter 2.

Die erfindungsgemäße Stanzvorrichtung 1 eignet sich zum Herausstanzen von Gewebeproben bei beliebigen Organismen bzw. Tieren. Als Versuchstiere kommen insbesondere Mäuse, Ratten oder Fische in Frage. Bei Fischen wird vorzugsweise eine Flosse des zu untersuchenden Fisches gemäß mit der erfindungsgemäßen Stanzvorrichtung 1 gestanzt. Da es sich bei der erfindungsgemäßen Stanzvorrichtung 1 vorzugsweise um ein Einmalwerkzeug handelt, entfällt auch die sonst notwendige Reinigung des Probeentnahmewerkzeuges. Eine Verschleppung von Gewebeproben ist bei der erfindungsgemäßen Stanzvorrichtung 1 ausgeschlossen. Die erfindungsgemäße Stanzvorrichtung 1 kann sowohl für den Bereich der Forschung als auch für die Tierzucht eingesetzt werden. Während des Stanzvorgangs kommt die herausgestanzte Gewebeprobe nicht mit der Hand der Bedienperson in Kontakt und die Kontamination der Bestandsprobe durch Verunreinigungen, die an der Hand der Bedienperson haften, wird hierdurch vermieden. Die erfindungsgemäße Stanzvorrichtung 1 ist besonders einfach zu bedienen und kann in jeder Lage eingesetzt werden. Die Zuordnung der Gewebeproben 3 zu den Versuchstieren wird erheblich erleichtert, da die Markierung und die Gewebeprobeentnahme in einem Arbeitsschritt erfolgen. Eine Amputation von Schwanzspitze, Zehen oder Flossen von Versuchstieren kann vermieden werden, so dass die erfindungsgemäße Stanzvorrichtung 1 auch dem Tierschutz dient. Eine Verunreinigung der in dem Aufnahmebehälter 2 vorabgefüllten Reaktionsflüssigkeit kann in jedem Fall vermieden werden.

Bezügszeichenliste
- 1: Stanzvorrichtung
- 2: Aufnahmebehälter
- 3: die herausgestanzte Gewebeprobe
- 4: Ohr
- 5: Behälterverschluss
- 6: Stanzöffnung
- 7: Bügel
- 8: Stanze
- 9: Reaktionsflüssigkeit
- 10: Halteeinrichtung
- 11: Druckfeder
- 12: Endstück mit Auswölbung
- 13: Sollbruchstelle
- 14: Ständer
- 15: Ständerplatte
- 16: Ständerplatte

## Patentansprüche

1. Vorrichtung zum Herausstanzen einer Gewebeprobe mit:
(a) einem Aufnahmebehälter (2) zur Aufnahme der Gewebeprobe;
(b) einem in den Aufnahmebehälter (2) einsetzbaren Behälterverschluss (5) zum Verschließen des Aufnahmebehälters (2),
wobei der Behälterverschluss (5) eine Stanzöffnung (6) aufweist, und mit
(c) einem mit dem Behälterverschluss (5) verbundenen Bügel (7), der eine in die Stanzöffnung (6) passgenau absenkbare Stanze (8) trägt.

2. Vorrichtung nach Anspruch 1
**dadurch gekennzeichnet, dass** der Behälterverschluss (5) und der Bügel (7) aus Kunststoff bestehen.

3. Vorrichtung nach Anspruch 1
**dadurch gekennzeichnet, dass** die Stanze (8) am distalen Ende des Bügels (7) angebracht ist und gegen eine Federkraft des Bügels (7) durch aufgebrachten mechanischen Druck in die Stanzöffnung (6) des Behälterverschlusses (5) absenkbar ist.

4. Vorrichtung nach einem der Ansprüche 1 bis 3 **dadurch gekennzeichnet, dass** eine Halteeinrichtung (10) vorgesehen ist, in die der durch den Behälterverschluss (5) verschlossene Aufnahmebehälter (2) eingehängt wird.

5. Vorrichtung nach Anspruch 4
**dadurch gekennzeichnet, dass** eine Halteeinrichtung (10) eine manuell betätigbare Druckfeder (11) aufweist, durch die mechanisch Druck auf den Bügel (7) aufbringbar ist.

6. Vorrichtung nach Anspruch 5
**dadurch gekennzeichnet, dass** die durch den Bügel (7) getragene und in die Stanzöffnung (6) passgenau abgesenkte Stanze (8) durch die Federkraft des Bügels (7) zur erneuten Betätigung von dem Behälterverschluss (5) zurückfedert, wenn der durch die Druckfeder (11) manuell aufgebrachte mechanische Druck innerhalb kürzester Zeit beendet wird.

7. Vorrichtung nach Anspruch 5
**dadurch gekennzeichnet, dass** die durch den Bügel (7) getragene und in die Stanzöffnung (6) passgenau abgesenkte Stanze (8) in dem Behälterverschluss (5) zu dessen Versiegelung stecken bleibt, wenn der durch die Druckfeder (11) manuell aufgebrachte mechanische Druck erst nach einer längeren Zeit beendet wird.

8. Vorrichtung nach Anspruch 7
**dadurch gekennzeichnet, dass** die durch den Bügel (7) getragene Stanze (8) über eine Sollbruchstelle (13) mit dem Bügel (7) verbunden ist.

9. Vorrichtung nach Anspruch 5
**dadurch gekennzeichnet, dass** die Druckfeder (11) der Halteeinrichtung (10) aus Metall besteht.

10. Vorrichtung nach Anspruch 5
**dadurch gekennzeichnet, dass** die Druckfeder (11) der Halteeinrichtung (10) aus Kunststoff besteht.

11. Vorrichtung nach Anspruch 1
**dadurch gekennzeichnet, dass** der Aufnahmebehälter (2) zylinderförmig ist, wobei die Stanzöffnung (6) des Behälterverschlusses (5) exzentrisch zu der Symmetrieachse (S) des Aufnahmebehälters (2) angeordnet ist.

12. Vorrichtung nach Anspruch 1
**dadurch gekennzeichnet, dass** der Aufnahmebehälter (2) transparent ist.

13. Vorrichtung nach Anspruch 1
**dadurch gekennzeichnet, dass** in den Aufnahmebehälter (2) eine Reaktionsflüssigkeit für die herausgestanzte Gewebeprobe (3) abgefüllt ist.

14. Vorrichtung nach Anspruch 13
**dadurch gekennzeichnet, dass** die Reaktionsflüssigkeit eine Enzymlösung ist.

15. Vorrichtung nach Anspruch 1
**dadurch gekennzeichnet, dass** der Aufnahmebehälter (2) ein Eppendorf-Gefäß ist.

## Claims

1. Device for punching out a tissue sample, with:
(a) a receiving container (2) for receiving the tissue sample,
(b) a container closure element (5) which can be inserted into the receiving container (2) in order to close said receiving container (2), the container closure element (5) having a punch aperture (6), and with
(c) a clip (7) which is connected to the container closure element (5) and carries a punch (8) that can be lowered with an exact fit into the punch aperture (6).

2. Device according to Claim 1, **characterized in that** the container closure element (5) and the clip (7) are made of plastic.

3. Device according to Claim 1, **characterized in that** the punch (8) is arranged at the distal end of the clip (7) and, by application of mechanical pressure, can be lowered into the punch aperture (6) of the container closure element (5) counter to an elastic force of the clip (7).

4. Device according to one of Claims 1 to 3, **characterized in that** a holding means (10) is provided in which the receiving container (2) closed by the container closure element (5) is suspended.

5. Device according to Claim 4, **characterized in that** a holding means (10) comprises a manually actuated compression spring (11) via which pressure can be applied mechanically to the clip (7).

6. Device according to Claim 5, **characterized in that** the punch (8) carried by the clip (7) and lowered with an exact fit into the punch aperture (6) springs back from the container closure element (5), by means of the elastic force of the clip (7), for renewed actuation if the mechanical pressure applied manually via the compression spring (11) is terminated within a very short time.

7. Device according to Claim 5, **characterized in that** the punch (8) carried by the clip (7) and lowered with an exact fit into the punch aperture (6) remains in the container closure element (5), in order to seal the latter, if the mechanical pressure applied manually via the compression spring (11) is terminated only after a longer time.

8. Device according to Claim 7, **characterized in that** the punch (8) carried by the clip (7) is connected to the clip (7) via a predetermined breaking point (13).

9. Device according to Claim 5, **characterized in that** the compression spring (11) of the holding means (10) is made of metal.

10. Device according to Claim 5, **characterized in that** the compression spring (11) of the holding means (10) is made of plastic.

11. Device according to Claim 1, **characterized in that** the receiving container (2) is cylindrical, the punch aperture (6) of the container closure element (5) being arranged eccentrically with respect to the axis of symmetry (S) of the receiving container (2).

12. Device according to Claim 1, **characterized in that** the receiving container (2) is transparent.

13. Device according to Claim 1, **characterized in that** a reaction fluid for the punched-out tissue sample (3) is introduced into the receiving container (2).

14. Device according to Claim 13, **characterized in that** the reaction fluid is an enzyme solution.

15. Device according to Claim 1, **characterized in that** the receiving container (2) is an Eppendorf tube.

## Revendications

1. Dispositif pour découper un échantillon de tissu avec :
(a) un récipient de prélèvement (2) pour recevoir l'échantillon de tissu ;
(b) un bouchon de récipient (5) pouvant être introduit dans le récipient de prélèvement (2) pour obturer le récipient de prélèvement (2), le bouchon de récipient (5) comportant une ouverture d'estampage (6) ; et
(c) une pince (7) qui est liée au bouchon de récipient (5) et qui porte une poinçonneuse (8) pouvant être abaissée avec précision dans l'ouverture d'estampage (6).

2. Dispositif selon la revendication 1, **caractérisé en ce que** le bouchon de récipient (5) et la pince (7) sont en matière plastique.

3. Dispositif selon la revendication 1, **caractérisé en ce que** la poinçonneuse (8) est placée à l'extrémité distale de la pince (7) et peut être abaissée dans l'ouverture d'estampage (6) du bouchon de récipient (5) en exerçant une pression mécanique contre une force élastique de la pince (7).

4. Dispositif selon l'une des revendications 1 à 3, **caractérisé en ce qu'**il est prévu un dispositif de saisie (10) dans lequel est accroché le récipient de prélèvement (2) obturé par le bouchon de récipient (5).

5. Dispositif selon la revendication 4, **caractérisé en ce qu'**un dispositif de saisie (10) comporte un ressort de pression (11) qui peut être actionné manuellement et par lequel une pression peut être exercée mécaniquement sur la pince (7).

6. Dispositif selon la revendication 5, **caractérisé en ce que** la poinçonneuse (8) portée par la pince (7) et pouvant être abaissée exactement dans l'ouverture d'estampage (6) est retirée du bouchon de récipient (5) par la force élastique de la pince (7) en vue d'un nouvel actionnement lorsque la pression mécanique exercée manuellement par le ressort de pression (11) est retirée après un temps très court.

7. Dispositif selon la revendication 5, **caractérisé en ce que** la poinçonneuse (8) portée par la pince (7) et pouvant être abaissée exactement dans l'ouverture d'estampage (6) reste enfoncée dans le bouchon de récipient (5) pour obturer celui-ci lorsque la pression mécanique exercée manuellement par le ressort de pression (11) est retirée après un temps plus long.

8. Dispositif selon la revendication 7, **caractérisé en ce que** la poinçonneuse (8) portée par la pince (7) est liée à la pince (7) par l'intermédiaire d'un point destiné à la rupture (13).

9. Dispositif selon la revendication 5, **caractérisé en ce que** le ressort de pression (11) du dispositif de saisie (10) est en métal.

10. Dispositif selon la revendication 5, **caractérisé en ce que** le ressort de pression (11) du dispositif de saisie (10) est en matière plastique.

11. Dispositif selon la revendication 1, **caractérisé en ce que** le récipient de prélèvement (2) est de forme cylindrique, l'ouverture d'estampage (6) du bouchon de récipient (5) étant agencé de façon excentrée par rapport à l'axe de symétrie (S) du récipient de prélèvement (2).

12. Dispositif selon la revendication 1, **caractérisé en ce que** le récipient de prélèvement (2) est transparent.

13. Dispositif selon la revendication 1, **caractérisé en ce que** le récipient de prélèvement (2) est rempli d'un liquide réactif pour l'échantillon de tissu découpé (3).

14. Dispositif selon la revendication 13, **caractérisé en ce que** le liquide réactif est une solution enzymatique.

15. Dispositif selon la revendication 1, **caractérisé en ce que** le récipient de prélèvement (2) est une éprouvette Eppendorf.
